(19) Europäisches Patentamt
European Patent Office
Office européen des brevets

(11) **EP 3 858 415 B1**

(12) **EUROPÄISCHE PATENTSCHRIFT**

(45) Veröffentlichungstag und Bekanntmachung des Hinweises auf die Patenterteilung:
**06.03.2024 Patentblatt 2024/10**

(21) Anmeldenummer: **21000006.3**

(22) Anmeldetag: **14.01.2021**

(51) Internationale Patentklassifikation (IPC):
***A61M 16/16*** *(2006.01)*

(52) Gemeinsame Patentklassifikation (CPC):
**A61M 16/16;** A61M 11/042; A61M 16/024;
A61M 16/1095; A61M 16/161; A61M 2016/0024;
A61M 2016/0039; A61M 2205/15; A61M 2205/3317;
A61M 2205/3365; A61M 2205/3368;
A61M 2205/3386; A61M 2205/3389; A61M 2205/42;
A61M 2205/505

(54) **VORRICHTUNG ZUR ANFEUCHTUNG VON ATEMGAS**

DEVICE FOR HUMIDIFYING RESPIRATORY GAS

DISPOSITIF D'HUMIDIFICATION DU GAZ RESPIRATOIRE

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorität: **28.01.2020 DE 102020101950**

(43) Veröffentlichungstag der Anmeldung:
**04.08.2021 Patentblatt 2021/31**

(73) Patentinhaber: **Löwenstein Medical Technology S.A.**
**2557 Luxembourg (LU)**

(72) Erfinder:
• **ALSHUT, Rüdiger**
**77880 Sasbach (DE)**
• **DÖMER, Benno**
**76275 Ettlingen (DE)**
• **SCHWAIBOLD, Matthias**
**76228 Karlsruhe (DE)**

(74) Vertreter: **Marx, Thomas**
**Löwenstein Medical Technology GmbH + Co. KG
IP Management
Kronsaalsweg 40
22525 Hamburg (DE)**

(56) Entgegenhaltungen:
EP-A1- 4 051 352         WO-A1-2004/020031
WO-A1-2016/139645        US-A1- 2015 165 146
US-A1- 2016 067 443      US-A1- 2016 193 437
US-A1- 2019 076 617      US-A1- 2019 298 962

**Beschreibung**

Eine typische Verwendung von Atemluftbefeuchtern erfolgt in Zusammenhang mit Atemluftversorgungen, die im Rahmen einer CPAP-Therapie (Continious-Positive-Airway-Pressure), Bilevel-, APAP- oder Heimbeatmung eingesetzt werden. Sie vermeiden ein Austrocknen der Atemwege insbesondere bei längeren Beatmungsphasen. Der Atemluftbefeuchter weist in der Regel einen befüllbaren Wassertank und ein Heizelement auf. Üblicherweise werden Atemluftbefeuchter zeitgleich mit dem Beatmungsgerät aktiviert. Dadurch stellt sich beim Patienten, nach kurzer Verzögerung, erwärmte und angefeuchtete Luft ein.

US 2016/0067443 A1 offenbart ein Atemunterstützungsgerät mit einem Befeuchter, einer beheizten Leitung und einer Steuereinheit, die den Befeuchter und die beheizte Leitung steuert, ohne dass Sensoren im Gasstrom erforderlich sind.

US 2019/0298962 A1 offenbart einen Befeuchter mit einer Trennwand, die den Innenraum des Befeuchters in eine Füllkammer und eine Befeuchtungskammer unterteilt und bei der das Atemgas einem vordefinierten Durchgangsweg folgt.

WO 2004/020031 A1 offenbart ein Atemunterstützungsgerät mit einem Befeuchter und einer beheizten Leitung sowie einer Steuerung, die Durchflussrate, Temperatur und Feuchtigkeit von Gasen ohne externe Sensoren bestimmt und das Atemgas entsprechend anpasst.

US 2016/0193437 A1 offenbart eine Steuerung für ein Beatmungsgerät mit einem Befeuchter, wobei die Steuerung als separate bzw. als vom Beatmungsgerät separierbare Einheit betrieben werden kann.

US 2019/0076617 A1 offenbart ein Atmungsunterstützungssystem mit einem Befeuchter und einer zugehörigen Heizung zur Abgabe von erhitzten und befeuchteten Atemgasen an einen Patienten.

WO 2016/139645 A1 offenbart einen Befeuchter zur Befeuchtung der Atemwege eines beatmeten Patienten. Beim Einschalten des Befeuchters kann ein spezieller Algorithmus zur Steuerung der Heizleistung ausgeführt werden. Dabei kann die Heizleistung in einer ersten Phase höher als in einer nachfolgenden zweiten Phase eingestellt werden.

In Abhängigkeit von der speziellen Therapie kann dem Patienten ein variabler oder konstanter Druck verabreicht werden, um Atemwegsverschlüsse zu verringern oder zu beseitigen oder um ein akutes oder chronisches Atemversagen zu behandeln.

Manche Patienten jedoch wissen zwar die Vorteile von erwärmter und angefeuchteter Luft zu schätzen, können jedoch eben wegen der erwärmten und angefeuchteten Luft nicht einschlafen.

Andere Patienten wiederum wünschen sofort, also mit Aufnahme der Benutzung des Beatmungsgerätes, erwärmte und angefeuchtete Luft. Ein Problem ist die Bereitstellung erwärmter und angefeuchteter Luft im Falle von Leckagen.

Aufgabe der vorliegenden Erfindung ist es daher, einen Atemluftbefeuchter bereitzustellen, bei dem die Erwärmung und Befeuchtung verbessert erfolgt.

Die Erfindung betrifft ein Beatmungsgerät und wird in dem unabhängigen Anspruch 1 definiert. Die abhängigen Ansprüche definieren bevorzugte Ausführungsformen.

Es wird auch offenbart, jedoch nicht beansprucht, ein Verfahren zur Vorgabe der Heizleistung eines Heizelementes bei einem Beatmungsgerät mit einem Anfeuchter wobei sensorisch zumindest ein Parameter des Atemgases erfasst wird und wobei die Vorgabe der Heizleistung zumindest teilweise basierend auf dem Parameter des Atemgases erfolgt.

Das Beatmungsgerät ist erfindungsgemäß auch dadurch gekennzeichnet, dass die Steuereinheit die Heizleistung des Heizelementes zumindest teilweise basierend auf zumindest einem der folgenden Parameter einstellt; einem Druck des Atemgasstroms; einem Fluss oder Volumen des Atemgasstroms, eine absichtliche Leckage; ein unbeabsichtigte Leckage; eine Atemfrequenz; ein inspiratorisches Atemvolumen; ein exspiratorisches Volumen; ein I: E-Verhältnis; Beginn und Ende der Inspiration; Beginn und Ende der Exspiration; ein Spitzenfluss während der Inspiration; ein Spitzenflusses während der Exspiration; Umgebungstemperatur; Wassertemperatur, Luftfeuchtigkeit; eine Ausgangsleistung oder Zielleistung der Heizleistung; eine Übertragungsfunktion für die Heizleistung; ein Volumen des Schlauches; ein Volumen der Benutzerschnittstelle; die Zeitdauer seit Therapiebeginn; Messwerte externer, drahtlos angebundener Sensoren, z. B. in einem Smartphone, oder anderweitiger externer Daten, z. B. Wetter-App im Smartphone.

Das Beatmungsgerät ist erfindungsgemäß auch dadurch gekennzeichnet, dass der Sensor den Fluss des Atemgases ermittelt und die Steuereinheit die Heizleistung des Anfeuchters entsprechend dem Fluss des Atemgases steuert.

Das Beatmungsgerät ist erfindungsgemäß auch dadurch gekennzeichnet, dass der Sensor den Fluss des Atemgases ermittelt und die Steuereinheit die Heizleistung des Anfeuchters entsprechend dem Fluss des Atemgases und einer hinterlegten Übertragungsfunktion für die Heizleistung steuert.

Das Beatmungsgerät ist erfindungsgemäß auch dadurch gekennzeichnet, dass die Steuereinheit einen absichtlichen Leckage-Fluss des Atemgases ermittelt und die Heizleistung des Anfeuchters entsprechend dem absichtlichen Leckage-Fluss des Atemgases steuert.

Das Beatmungsgerät ist erfindungsgemäß auch dadurch gekennzeichnet, dass die Steuereinheit einen unabsichtlichen Leckage-Fluss des Atemgases ermittelt und die Heizleistung des Anfeuchters entsprechend dem unabsichtlichen Leckage-Fluss des Atemgases steuert.

Das Beatmungsgerät ist erfindungsgemäß auch dadurch gekennzeichnet, dass die Steuereinheit einen mittleren Gesamtfluss des Atemgases ermittelt und die Heizleistung des Anfeuchters entsprechend dem mittleren Gesamtfluss des Atemgases steuert.

Das Beatmungsgerät ist erfindungsgemäß auch dadurch gekennzeichnet, dass die Heizleistung des Anfeuchters basierend auf dem mittleren Gesamtfluss derart gesteuert wird, dass die absolute Feuchte (Wassermenge pro Volumen) des abgegebenen Atemgases annähernd konstant bleibt.

Das Beatmungsgerät ist erfindungsgemäß auch dadurch gekennzeichnet, dass die Heizleistung gesteuert wird, um die Austrocknung der Schleimhäute des Patienten bei hoher Leckage zu reduzieren und gleichzeitig zu verhindern, dass bei niedriger Leckage Wassertropfen im Beatmungsschlauch auskondensieren.

Das Beatmungsgerät ist erfindungsgemäß auch dadurch gekennzeichnet, dass die Steuereinheit die Heizleistung des Anfeuchters so steuert, dass über einen Flussbereich vom 1 l/min bis 300 l/min ein konstantes Feuchtigkeitsniveau in dem angefeuchteten Atemgas erreicht wird.

Das Beatmungsgerät ist erfindungsgemäß auch dadurch gekennzeichnet, dass der vom Beatmungsgerät abgegebene Fluss über einen Sensor gemessen oder über ein indirektes Verfahren ermittelt wird beispielsweise aus dem gemessenen Druck und der Drehzahl des Gebläses.

Anschließend wird dieser Fluss von der Steuereinheit gemittelt, vorzugsweise über mindestens einen Atemzug oder über x (z.B. 2) Sekunden und unabhängig von der erkannten Atemphase, damit eventuelle Triggerfehler keine Auswirkung auf die Anfeuchter-Regelung haben.

Erfindungsgemäß kann auch auf die digitale Mittelung verzichten werden und der Heizstab mit sehr dynamisch schwankender Leistung betrieben werden - das Wasser wirkt ja ohnehin als Tiefpass. Insofern hat die Mittelung technische Vorteile, da die Spitzenlast für Netzteil, Spannungsregler, Heizstab etc. sinkt.

Das Beatmungsgerät ist erfindungsgemäß auch dadurch gekennzeichnet, dass die Heizleistung stufenlos anhand eines Kennfeldes, welches die benötigte Leistung bei bestimmten mittleren Gesamtflüssen aufschlüsselt, verändert wird.

Das Beatmungsgerät ist erfindungsgemäß auch dadurch gekennzeichnet, dass mehrere diskrete Stufen Heizleistung vorgebbar sind, wobei jeweils zumindest eine Kennlinie pro Stufe der Heizleistung abrufbar hinterlegt ist.

Das Beatmungsgerät ist erfindungsgemäß auch dadurch gekennzeichnet, dass die die Steuerung der Heizleistung Korrektur-Parameter hinterlegt sind, um Umgebungstemperaturen oder Luftfeuchte oder Wasser-Einfülltemperaturen auszugleichen.

Erfindungsgemäß wird auch die absolute Feuchte gemessen. Dies dient insbesondere zur Vermessung des Kennfeldes. Erfindungsgemäß könnten auch noch Kennfelder oder Korrektur-Parameter hinterlegt werden, um Umgebungstemperaturen / Luftfeuchte / Wasser-Einfülltemperaturen etc. auszugleichen. Die Kennfelder werden dann von der Steuerung ausgelesen und angewendet.

Das Beatmungsgerät ist erfindungsgemäß auch dadurch gekennzeichnet, dass zusätzlich eine Schlauchheizung umfasst ist, wobei die Schlauchheizung in Abhängigkeit vom Fluss oder Gesamtfluss oder von der Leckage gesteuert wird.

Das Beatmungsgerät ist erfindungsgemäß auch dadurch gekennzeichnet, dass der Anfeuchter bei der Verbindung mit dem Beatmungsgerät mit der Elektronik des Beatmungsgerätes gekoppelt und über das Beatmungsgerät gesteuert wird.

Das Beatmungsgerät ist erfindungsgemäß auch dadurch gekennzeichnet, dass das Rampenmodul die Temperatur im Wasserbehälter und/oder die Umgebungstemperatur für die Steuerung der Heizleistung zumindest in der ersten Phase berücksichtigt und dazu Mittel zum Erfassen der Wassertemperatur im Anfeuchter angeordnet sind, um der Steuereinheit eine IST-Temperatur zu übermitteln.

Wenn eine mittlere oder niedrige Anfeuchterstufe eingestellt ist, erwärmt sich der Heizstab sehr langsam. Der konstante Arbeitspunkt wird erst nach 30-90 Minuten erreicht, so lange dauert die Aufwärmphase. Das fühlt sich dann für den Patienten beim Einschlafen so an, als wäre der Anfeuchter gar nicht eingeschaltet. Erfindungsgemäß sorgt nun das Rampenmodul, zum Beispiel über die Funktion des Temperaturanstiegs, dafür, dass für die ersten x (1 - 30) Minuten immer eine sehr hohe / die höchste Heizleistung verwendet wird, damit der stabile Arbeitspunkt rasch erreicht wird. Bei niedriger eingestellter Stufe der Anfeuchtung also für eine kurze Zeit, bei mittlerer Stufe für eine längere Zeit. Damit erreicht der Heizstab nach wenigen Minuten seine Zieltemperatur. Die Steuerung gibt beispielsweise x Minuten Rampenmodul mit höchster Leistung für jede eingestellte Stufe der Anfeuchtung vor. Erfindungsgemäß sind auch intelligentere Aufwärmphasen vorgesehen, die z. B. die Temperatur des eingefüllten Wassers oder der Umgebung berücksichtigen.

Das Beatmungsgerät ist erfindungsgemäß auch dadurch gekennzeichnet, dass das Rampenmodul unterschiedliche Leistungskurven abrufbar oder einstellbar vorgeben kann. Das Beatmungsgerät ist erfindungsgemäß auch dadurch gekennzeichnet, dass das Rampenmodul zusätzlich ein Verzögerungsmodul beinhaltet, welches die Steuereinheit zu einer einstellbar verzögerten Vorgabe der Heizleistung an das Heizelement veranlasst.

Das Beatmungsgerät ist erfindungsgemäß auch dadurch gekennzeichnet, dass das Verzögerungsmodul die Steuereinheit zu einem einstellbar verzögerten Beginn einer Beatmungsfunktion veranlasst.

Das Beatmungsgerät ist erfindungsgemäß auch dadurch gekennzeichnet, dass das Verzögerungsmodul eine konkrete Zeitvorgabe in Minuten ermöglicht.

Das Beatmungsgerät ist erfindungsgemäß auch dadurch gekennzeichnet, dass die Leistung durch Pulsweitenmodulation nachgeregelt wird, damit auch bei einem sich ändernden Widerstand des Heizelementes eine gleichbleibende Leistungsabgabe ermöglicht wird.

Das Beatmungsgerät ist erfindungsgemäß auch dadurch gekennzeichnet, dass das Heizelement eine temperaturabhängige Widerstandskennlinie aufweist und im Beatmungsgerät der aktuelle Widerstandswert aus dem gemessenen Strom und der Spannung am Heizelement errechnet wird.

Das Beatmungsgerät ist erfindungsgemäß auch dadurch gekennzeichnet, dass der aktuelle Widerstandswert mit Schwellwerten verglichen wird zur Erkennung eines leeren Wasserbehälters.

Das Beatmungsgerät ist erfindungsgemäß auch dadurch gekennzeichnet, dass bei Überschreitung eines Schwellwertes die Steuereinheit, die Heizleistung ausgeschaltet und eine Signalisierung dieses Zustands an den Anwender erfolgt.

Das Beatmungsgerät ist erfindungsgemäß auch dadurch gekennzeichnet, dass Die Erfindung ist auch dadurch gekennzeichnet, dass die Steuereinheit die tatsächlich an das Heizelement abgegebene Leistung speichert und die Werte an mindestens einer Schnittstelle bereitstellt.

Das Beatmungsgerät ist erfindungsgemäß auch dadurch gekennzeichnet, dass das Rampenmodul in einer ersten, kürzer andauernden Phase eine hohe oder die höchste Heizleistung, steuert als in einer zweiten, längeren Phase und wobei der Sensor den Fluss des Atemgases ermittelt und die Steuereinheit die Heizleistung des Anfeuchters zumindest in der zweiten, längeren Phase entsprechend dem Fluss des Atemgases steuert.

Die Erfindung ist auch dadurch gekennzeichnet, dass die Steuereinheit deaktivierbar ist und/oder eine feste Heizleistung vorgibt.

Das Beatmungsgerät ist erfindungsgemäß auch dadurch gekennzeichnet, dass das Heizelement (13) eine temperaturabhängige Widerstandskennlinie aufweist und im Beatmungsgerät (1) der aktuelle Widerstandswert aus dem gemessenen Strom und der Spannung am Heizelement (13) errechnet wird, wobei der aktuelle Widerstandswert mit Schwellwerten verglichen wird zur Erkennung eines leeren Wasserbehälters und wobei bei Überschreitung eines Schwellwertes die Steuereinheit (16) die Heizleistung ausschaltet.

Das erfindungsgemäße Verfahren ist ebenfalls dadurch gekennzeichnet, dass der Parameter des Atemgases der Fluss des Atemgases ist, wobei die Heizleistung für einen ersten Zeitraum x von 1 bis 30 Minuten mindestens 75% der Maximalleistung beträgt und/oder das Heizelement nach einer einstellbaren Zeit verzögert nach Beatmungsbeginn hinzugeschalten wird.

Erfindungsgemäß gibt die Steuereinheit die Heizleistung des Heizelementes zumindest teilweise oder zeitweise basierend auf folgender Formel vor:

$$H = c_{PL}(\theta - 0°C)m_L + \big(r_0 + c_{PD}(\theta - 0°C)\big)m_W$$

wobei $c_{PL}$ die spezifische Wärmekapazität von Luft ($1{,}0\ \frac{kJ}{kg \cdot K}$), $\theta$ die Zieltemperatur, $r_0$ die Verdampfungsenthalpie von Wasser ($2{,}6\ \frac{MJ}{kg \cdot K}$) und $c_{PD}$ die spezifische Wärmekapazität vom Wasserdampf ($1{,}9\ \frac{kJ}{kg \cdot K}$) ist. Der Term ($\theta$-0°C) kann auch als $\Delta$ beschrieben werden.

Beispielsweise kann von einem Beatmungsgerät ein kontinuierlicher positiver Atemwegsdruck (CPAP) während des gesamten Atemzyklus des Patienten bereitgestellt werden. Bi-Level-Überdruck der Atemwege (Bi-PAP) kann in Abstimmung mit den Einatmungs- und Ausatmungsbemühungen des Patienten mindestens zwei verschiedene Drücke bereitstellen. In anderen Systemen können Auto-PAP-Systeme (Auto-Titration Positive Airway Pressure) den Therapiedruck basierend auf dem Grad der Atemunterstützung regulieren, den der Patienten an einem bestimmten Punkt während eines Atems benötigt. Die Erfindung kann auch bei klinischen Beatmungsgeräten, Säuglingsbeatmungsgeräten oder Highflow-Geräten eingesetzt werden.

Unabhängig von der jeweiligen Therapie umfassen diese Beatmungssysteme typischerweise mindestens eine Atemgaseinheit als Gebläse- oder Ventil-einheit und eine Benutzerschnittstelle oder -maske. Ein Zufuhrschlauch kann die Atemgaseinheit mit der Maske verbinden, wobei der Schlauch und die Maske zusammen eine Gaszufuhrleitung zwischen der Atemgaseinheit und dem Benutzer definieren.

Die Maske kann so konfiguriert sein, dass sie relativ zum Kopf des Benutzers so befestigt ist, dass sie eine im Allgemeinen luftdichte Abdichtung mit dem Atemweg des Benutzers bildet (z. B. Abdichtung um das Gesicht, die Nasenlöcher und / oder den Mund). Infolgedessen kann die Atemgaseinheit einen Druckgasstrom erzeugen, der über den Schlauch in den Atemweg abgegeben wird.

Ein Anfeuchter zum Befeuchten des von der Atemgaseinheit gelieferten Gases kann ebenfalls vorgesehen sein. Anfeuchter umfassen typischerweise ein beheiztes Wasserreservoir, das ein Wasservolumen mit einer relativ großen Oberfläche enthält. Das Reservoir befindet sich zwischen der Atemgaseinheit und der Maske. Das Atemgas aus der Atemgaseinheit kann über das Wasserreservoir geleitet werden, in dem sich das erwärmte Wasser befindet. Das Atemgas nimmt dabei Feuchtigkeit auf und wird dann über den Schlauch dem Patienten an der Schnittstelle zur Verfügung gestellt.

Klimaregelung: Der Anwender stellt ein Leistungsniveau für den Anfeuchter gemäß seinem optimalen Atemkomfort ein, welches sich auf ein bestimmtes Leckageniveau bezieht, bevorzugt bei dichter Maske (nur Spülflow). Wenn nun die Leckage steigt, wird anhand einer hinterlegten Funktion, z. B. Gerade, oder anhand eines Kennfeldes die Leistung des Anfeuchters erhöht, da eine größere Menge Luft pro Zeiteinheit angefeuchtet werden muss und insbesondere bei Mundleckagen durch den erhöhten Luftstrom auch eine größere Gefahr für die Austrocknung der Schleimhäute besteht. Durch die Kopplung der Regelung an die Leckage (mittlerer Luftstrom) sparen wir uns zusätzliche Sensoren wie einen Feuchtesensor an der Maske oder im Schlauch. In einer bevorzugten Ausführung wird zusätzlich zum Anfeuchter auch die Leistung der Schlauchheizung nach derselben Logik, lediglich anderer Parametrierung geregelt, da auch eine erhöhte Menge Luft erwärmt werden muss bei Leckage.

Der Atemluftbefeuchter weist beispielsweise eine kompakte Gerätekonstruktion auf, die dem Konturverlauf des Beatmungsgerätes angepasst ist. Der Atemluftanfeuchter besteht aus einem Gehäuseunterteil mit Heizelement, einem Gehäuseoberteil und einem Multifunktionsmittelteil aus Silikon mit einer Dichtauflage zum Gehäuseunterteil und Gehäuseoberteil.

In den folgenden Zeichnungen sind Ausführungsbeispiele der Erfindung dargestellt. Es zeigen:

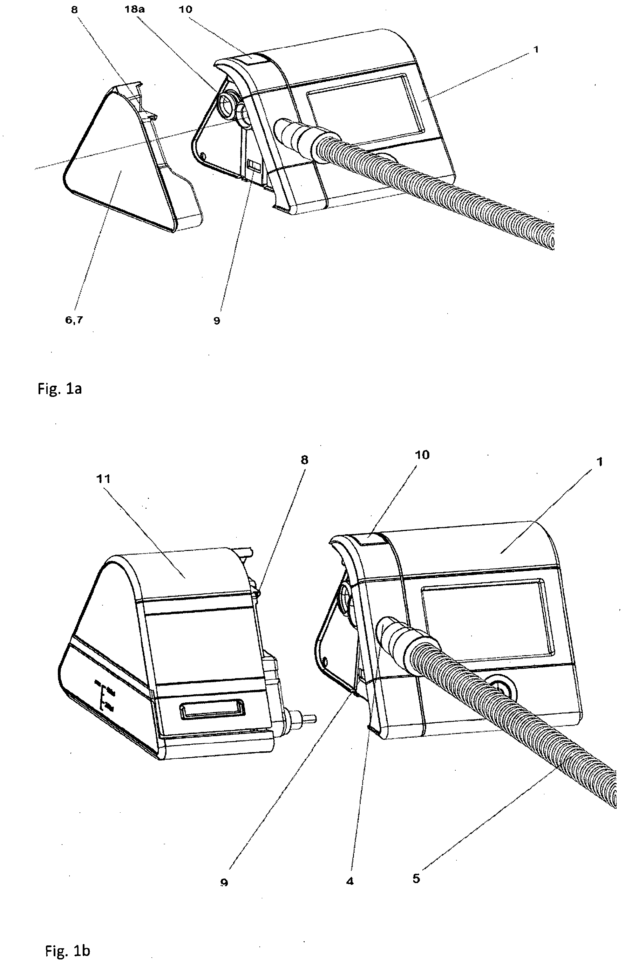

Fig. 1a und 1b zeigen ein den grundsätzlichen Aufbau eines Beatmungsgerätes, welches ohne und mit Atemluftanfeuchter ausgeführt ist. Das Beatmungsgerät (1) weist ein Bedienfeld (2) sowie eine Anzeige beispielsweise mit Touchscreen (3) auf. Das Beatmungsgerät ist darüber hinaus im Geräteinnenraum mit einer Fördereinrichtung für das Atemgas z.B. in Form einer Atemgaspumpe, einem Gebläse oder einem Ventilsystem sowie einer Steuerung für den elektrischen Antrieb ausgestattet. Über ein Anschlusselement (4) wird ein Beatmungsschlauch (5) angeschlossen an dessen Ende sich die Schnittstelle für den Patienten beispielsweise in Form einer Beatmungsmaske oder Beatmungsbrille befindet.

Um den gewünschten Druckgasstrom in dem Schlauch bereitzustellen, kann das Gebläse Teil eines Strömungsgenerators sein, der ein Gebläsegehäuse aufweist, das ein Gebläserad oder einen Ventilator enthält. Ein Elektromotor, beispielsweise ein bürstenloser Gleichstrommotor, kann das Gebläserad oder den Ventilator während des Gebrauchs drehen. Wenn sich der Lüfter dreht, saugt er Atemgas (z. B. Umgebungsluft) über einen Einlass des Gebläses an, wo das Gas dann durch den Lüfter komprimiert und durch den Auslass ausgestoßen wird. Durch Steuern der Drehzahl des Gebläses kann der Druck des Gases gesteuert werden, um dem Patienten den gewünschten Behandlungsdruck bereitzustellen.

Das Beatmungsgerät kann ferner eine Steuerung enthalten, die unter anderen Aufgaben eine Drehzahl des Motors modulieren oder auf andere Weise steuern kann, um beispielsweise eine Variable Durchflussmenge bei konstantem Druck zu erzeugen. Die Steuerung kann in einer Ausführungsform eine mikroprozessorbasierte Motorsteuerung enthalten. Alle elektrischen Komponenten können entweder von einer Batterie und/oder einer Wechselstrom- oder Gleichstromquelle über ein elektrisches Kabel gespeist werden. Die Steuerung kann andere Komponenten des Systems, wie hierin weiter beschrieben, elektrisch miteinander verbinden z.B. Anfeuchter und Beatmungsgerät.

Das Beatmungsgerät kann ferner einen Drucksensor enthalten. In einer Ausführungsform befindet sich der Drucksensor innerhalb des Gehäuses. Beispielsweise befindet sich in der dargestellten Ausführungsform der Drucksensor innerhalb des Gehäuses und ist an oder in der Nähe des Auslasses über eine angeschlossen. Der Drucksensor kann ein elektrisches Signal erzeugen, das proportional zu dem tatsächlichen gemessenen Druck ist. Das Drucksignal kann dann über eine elektrische Signalleitung Steuerung übertragen werden. Wie beschrieben, kann die Steuerung das Drucksignal mit einem gewünschten Druck vergleichen und nötigenfalls die Motordrehzahl an den Motor modulieren, um den gewünschten Druck einzustellen. Somit kann ein gewünschter Druck in dem Schlauch / der Maske ungeachtet der erwarteten Schwankungen des Flusses aufrechterhalten werden.

In anderen Ausführungsformen können auch zusätzliche Sensoren vorgesehen sein, z. B. ein Pneumotachometer oder Fluss-Sensor. Der Pneumotachometer kann die Steuerung mit einem elektrischen Signal versorgen, das proportional zu dem momentanen Fluss ist. Andere Sensoren, z. B. Temperatur, Feuchtigkeit usw., können ebenfalls vorgesehen sein und elektrisch mit der Steuerung verbunden sein.

Obwohl als Pneumotachometer beschrieben, können auch andere Vorrichtungen und / oder Verfahren zum Messen oder Schätzen des Luftstroms verwendet werden. Beispielsweise können andere Ausführungsformen eine Drehzahl des Motors oder die Spannung, den Strom oder die Leistungsaufnahme des Motors analysieren.

Um den Anfeuchter (11) an das Beatmungsgerät (1) zu koppeln wird die Seitenwand (6) des Beatmungsgerätes (1), die einen integrierten Schalldämpfer (7) aufweist und mit zwei Schnapphaken (8) in Aufnahmen (9) im Beatmungsgerät (1) verrasten, gelöst und gegen den Anfeuchter (11) ausgetauscht. Hierzu muss nur die Entriegelung (10) am Beatmungsgerät (1) betätigt werden. Durch Betätigen der Entriegelung (10) werden die Aufnahmen (9), die auf einer Verbindungsschiene miteinander verbunden, angeordnet sind, verschoben und die Verbindung zu den Schnapphaken (8) gelöst. Der Anfeuchter (11) weist ebenfalls Schnapphaken (8) auf, die in die gleichen Aufnahmen (9) des Beatmungsgerätes (1) greifen.

Das Gehäuseunterteil (12) weist eine Öffnung für das Heizelement (13) auf, das seitlich in die Öffnung das Gehäuseunterteil (12) eingeschraubt und mit einem O-Ring abgedichtet wird. Beim Anbau des Anfeuchters (11) an das Beatmungsgerät (1) wird das Heizelement (13) mit der Elektronik des Beatmungsgerätes (1) gekoppelt und über das Beatmungsgerät (1) gesteuert.

Der Innenraum des Gehäuseunterteiles (12) dient zur Aufnahme des Wassers, wobei der Wasserstand über Füllstandanzeigen abgelesen werden kann.

Die Steuerung des Anfeuchters erfolgt über die Regelung des Beatmungsgerätes. Hierbei können verschiedene Betriebszustände realisiert werden. Die Heizung des Anfeuchters kann vor Betrieb des Beatmungsgerätes vorheizen. Alternativ kann die Heizung des Anfeuchters verzögert, also nach Beginn der Beatmung, zugeschaltet werden. Zum anderen können verschiedene Leistungskurven realisiert werden. Beispielsweise in Form einer Rampe, wobei die Leistung zuerst sehr niedrig gehalten wird und dann in einem Sprung zu einer hohen Leistung ansteigt oder aber linear ansteigend ausgeführt sein kann. Ebenso ist die Vorwahl verschiedener Heizstufen möglich.

Die Heizstufen können mittels Leistungsregelung realisiert werden. Dabei wird durch eine zyklische Messung Strom und Spannung am Heizelement (13) erfasst und z.B. durch eine Pulsweitenmodulation nachgeregelt, damit auch bei einem sich ändernden Widerstand des Heizelementes eine gleichbleibende Leistungsabgabe ermöglicht wird.

Die Steuerung kann Daten analysieren, um verschiedene Parameter zu bestimmen, die dem Betrieb des Beatmungsgerätes zugeordnet sind. Zum Beispiel Atemfrequenz, Atemzugvolumen, absichtlicher Leckstrom, Gasstrom, Maskenleckstrom, inspiratorische / exspiratorische Übergänge, Vorhersagen bezüglich des Einsetzens und / oder der Dauer von Inspirationsphasen nachfolgender (zukünftiger) Atemzyklen, Betriebsparameter des Beatmungsgerätes oder des Anfeuchters. Noch weitere Parameter, die den Betrieb betreffen sind z. B. das Volumen (z. B. Länge und Durchmesser) des Schlauchs, das beabsichtigte Maskenleck, der Maskentotraum und der gewünschte Befeuchtungsgrad (vom Benutzer vorgegeben). Einige dieser Parameter können explizit eingegeben werden, während andere aus Konfigurationen ausgewählt werden können.

Anstatt beispielsweise explizite Abmessungen für den Schlauch und / oder die Maske einzugeben, muss der Benutzer möglicherweise nur eine Schlauch- oder Maskenteilnummer auswählen. Die Steuerung kann dann automatisch ein beabsichtigtes Leck, Schlauchvolumen und andere Parameter bestimmen.

In einer Konfiguration wie der von Fig. 1 ist beispielsweise ein relativ großes Wasservolumen mit einer entsprechend großen Oberfläche vorgesehen, damit der von dem Gebläse erzeugte Druckgasstrom den gewünschten Dampfgehalt auffangen kann, wenn er über das Wasser in dem Reservoir strömt. Beispielsweise kann ein Anfeuchter eine Reservoirgröße von 200 bis 800 ml haben und durchschnittlich 40 bis 500 Watt benötigen, um ein gewünschtes Feuchtigkeitsniveau zu liefern.

Der Anfeuchter kann ein Heizelement enthalten, das dazu dient, ein in dem Reservoir enthaltenes Wasservolumen auf einen gewünschten Punkt zu erwärmen, an dem das Wasser zumindest teilweise verdampft. Eine starre oder flexible Leitung kann einen Ausgang des Gebläses des Beatmungsgerätes mit einem Einlass des Reservoirs des Anfeuchters verbinden, während ein Schlauch typischerweise einen Auslass des Reservoirs mit einem Einlass der Maske verbindet. Der Anfeuchter kann, wie oben beschrieben, auch unmittelbar an das Beatmungsgerät adaptierbar sein.

Der Schlauch oder die Maske oder ein Zwischenstück kann eine Abluftöffnung aufweisen. Durch diese Abluftöffnung kann ein gewollter/absichtlicher Leckagefluss dauerhaft abströmen, um Ausatemgase abzuleiten. Ein durch das Gebläse erzeugter Druckgasstrom kann durch das Anfeuchtungsreservoir strömen, wobei verdampfende Feuchtigkeit darin mitgerissen wird, um einen Strom von befeuchtetem Atemgas zu erzeugen, der schließlich von dem Schlauch zu der Maske und zu dem Patienten geführt wird.

Die Benutzerschnittstelle oder Maske ist allgemein dargestellt, umfasst jedoch so gut wie jede Schnittstelle, die einen Patienten effektiv abdichtet (z. B. das Gesicht (Nase und / oder Mund) oder innerhalb der Nasenlöcher), so dass ein Fluss von Druckgas entsteht, der an die Benutzerschnittstelle abgegeben wird. Zum Beispiel könnte die Benutzerschnittstelle eine Gesichtsmaske sein, die den Mund und / oder die Nase des Benutzers bedeckt; ein Nasenlochkissen; oder eine Kombination solcher Masken. Der Einfachheit halber kann die Benutzerschnittstelle im Folgenden ohne Einschränkung auch als "Maske" bezeichnet werden.

Der Schlauch und die Benutzerschnittstelle (Maske) können zusammen eine Zuführungsleitung definieren, die einen Durchgang bildet, der den Druckgasstrom vom Auslass des Gebläses zum Anfeuchter und weiter zum Einlass der Maske und dann zum Atemweg transportiert oder auf andere Weise mit dem Patienten kommuniziert. Die Zufuhrleitung insgesamt kann eine oder mehrere Abluftöffnungen enthalten. Es ist bekannt, dass solche Entlüftungsöffnungen ein sogenanntes "beabsichtigtes oder gewünschtes Leck" bereitstellen. Ein beabsichtigtes Leck wird bereitgestellt, um das Spülen von Kohlendioxid (mit dem Ausatemgas) aus der Zufuhrleitung während der Ausatmungsphase jedes Atemzyklus zu unterstützen. In einer Ausführungsform können eine oder mehrere Entlüftungsöffnungen enthalten sein. Der beabsichtigte oder gewünschte Leckagefluss ist bekannt oder kann für bestimmte Flüsse und Drücke berechnet werden.

Die automatische Erkennung eines leeren Wasserbehälters wird ermöglicht, indem das Heizelement (13) über eine temperaturabhängige Widerstandskennlinie verfügt und im Beatmungsgerät (1) der aktuelle Widerstandswert aus dem gemessenen Strom und der Spannung am Heizelement (13) errechnet und mit Schwellwerten verglichen wird. Bei einem leeren Wasserbehälter wird das Heizelement (13) eine höhere Temperatur erreichen als bei noch vorhandenem Wasser. Bei Überschreitung des Schwellwerts kann die Heizleistung ausgeschaltet werden und eine Signalisierung dieses Zustands an den Anwender erfolgen. Wenn die Heizung des Anfeuchters bereits vor Betrieb des Beatmungsgerätes vorheizt, muss sichergestellt werden, dass es nicht zu Auskondensation des Wassers außerhalb des Anfeuchters kommt. Dies kann verhindert werden, indem im Beatmungsgerät eine Zeitbegrenzung der Vorheizzeit realisiert wird. Sofern innerhalb dieser Zeit der Betrieb des Beatmungsgeräts nicht aktiviert wird, kann das Vorheizen automatisch abgebrochen werden. In manchen Ausführungsformen kann die Erkennung so fein eingestellt sein, dass bereits eine nicht vollständige Bedeckung des Heizelements mit Wasser über die erreichte Temperatur und/oder den Widerstand des Heizelements erkannt werden kann.

In Heizpausen, also wenn eine Erwärmung des Wassers nicht stattfindet und/oder das Heizelement nicht erwärmt wird, ist es in manchen Ausführungsformen auch möglich, dass über den Widerstand des Heizelements die Wassertemperatur bestimmt werden kann. Beispielsweise kann die Wassertemperatur anhand einer Temperatur-Widerstands-Kennlinie des Heizelements ermittelt werden. Die ermittelte Wassertemperatur kann dann beispielsweise von der Steuereinheit verwendet werden um abzuschätzen ob beziehungsweise wann wieder geheizt werden kann und/oder muss.

In einer alternativen und/oder erweiterten Ausführungsform der Erfindung kann anhand der ermittelten Temperatur des Wassers während der Heizpausen sowie der zwischen zwei Heizpausen durch das Heizelement eingebrachten Energie (beispielsweise berechnet aus Spannung und Stromstärke sowie verschiedener Materialkonstanten und Größen des Heizelements) ein zumindest grober Rückschluss auf den Füllstand errechnet werden und gegebenenfalls auch auf einer Anzeige ausgegeben werden. Der grobe Rückschluss kann beispielsweise in Stufen wie "voll", "halbvoll" und "leer" oder auch als Füllanteil (100% bis 65%, 65% bis 35%, unter 35%) bestimmt werden.

Beispielsweise umfasst das Beatmungsgerät und/oder der Anfeuchter beziehungsweise die Steuerung/Steuereinheit ein Rampenmodul, welches eine Funktion des Temperaturanstiegs beinhaltet. Über diese Funktion kann zum Beispiel in den ersten x (1 bis 30) Minuten eine sehr hohe / die höchste Heizleistung (beispielsweise mindestens 75% der maximalen Leistung) verwendet werden, damit ein stabiler Arbeitspunkt schnell erreicht wird. Je nach gewünschter/eingestellter Stufe der Anfeuchtung kann beispielsweise die Dauer der hohen Heizleistung wahlweise manuell oder automatisch angepasst werden. Ist beispielsweise eine niedrige Anfeuchtungsstufe eingestellt, so wird für einen kürzeren Zeitraum mit hoher/maximaler Leistung geheizt als es für höhere Anfeuchtungsstufen eingestellt wäre. Erfindungsgemäß sind auch intelligentere Aufwärmphasen vorgesehen, die z. B. die Temperatur des eingefüllten Wassers oder der Umgebung berücksichtigen.

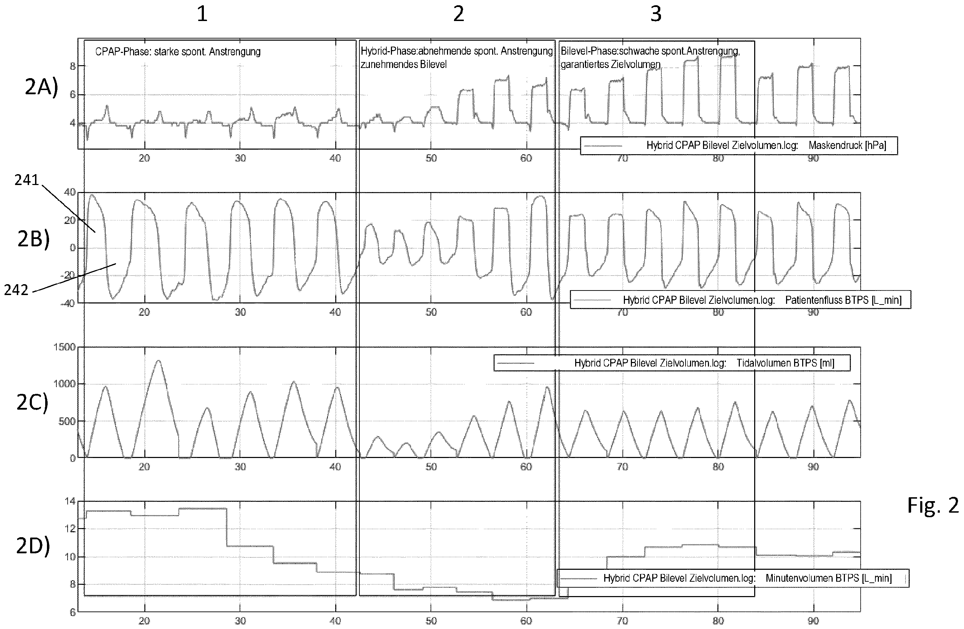

Fig. 2 zeigt die Auffeuchtung bzw. Anfeuchtung des Atemgases an dem Patienteninterface (hier Maske) in g/m3. In dem oberen Teil der Darstellung ist eine obere Aufzeichnung (31) zu erkennen, hier bleibt die Feuchte des Atemgases im Bereich 10 -11 g/m3 relativ konstant. Diese obere Aufzeichnung weist die erfindungsgemäße Vorgabe der Heizleistung des Heizelementes (13) zumindest teilweise basierend auf dem Parameter des Atemgases, nämlich basierend auf dem Fluss oder der Leckage oder dem mittleren Gesamtfluss. Zu dem mit einem Pfeil markierten Zeitpunkt wird die Vorgabe der Heizleistung des Heizelementes (13) zumindest teilweise basierend auf dem Parameter des Atemgases ausgeschaltet. In der Folge sinkt die Feuchte des Atemgases zunächst auf 8 und dann auf 6 g/m3 ab.

Die untere Aufzeichnung (32) zeigt den Verlauf der Feuchte des Atemgases bei einer die Vorgabe der Heizleistung des Heizelementes (13) ohne Berücksichtigung von Parametern des Atemgases. Hier sinkt die Feuchte des Atemgases von 10 auf 5 g/m3.

Im unteren Teil der Abbildung ist der Leckage-Fluss in l/min zeitsynchron mit dem oberen Verlauf der Feuchte des Atemgases dargestellt. Man erkennt, dass bei einem Anstieg der Leckage die Feuchte des Atemgases in der unteren Aufzeichnung (32) (ohne die erfindungsgemäße Fluss-Kompensation) deutlich einbricht.

Die Steuerung kann konfiguriert sein, um einfach eine Rate des Flusses (Fluss oder der Leckage oder dem mittleren Gesamtfluss) von Druckgas zu überwachen und die elektrische Leistung an das Heizelement während des Atemzyklus automatisch zu modulieren. Dieser Prozess kann die Menge an feuchtem, erwärmten Dampf modulieren, die proportional zum Druckgasstrom hinzugefügt wird, um ein im Wesentlichen konstantes Zielfeuchtigkeitsniveau im Druckgasstrom (selbst wenn sich der Druckgasstrom ändert) während des Atemzyklus und während der Behandlung aufrechtzuerhalten. Die Steuerung kann auch aktuelle oder frühere Atemzyklen analysieren, um zukünftige Atem- / Anfeuchtungserfordernisse vorherzusagen, und die Vorgabe für das Heizelement anzupassen.

In manchen beispielhaften Ausführungsformen erfolgt die Steuerung anhand hinterlegter Kennfelder, in denen eine benötigte Heizleistung des Heizelements mit dem Fluss des Atemgases und/oder einem Leckage-Fluss in Relation gesetzt sind.

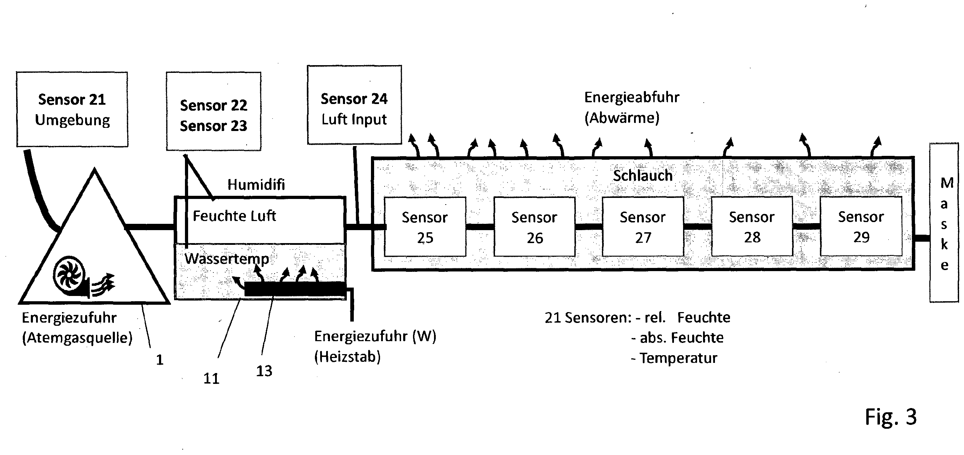

Fig. 3 zeigt Beatmungsgerät mit einer Atemgaseinheit (14) mit einem Anfeuchter (11), der ein Heizelement (13) und einen Wasserbehälter aufweist und zur Ankopplung an das Beatmungsgerät (1) ausgebildet ist, aufweisend: zumindest einen Sensor der Parameter des Atemgases erfasst und zumindest eine Steuereinheit (16), zur Vorgabe der Heizleistung des Heizelementes (13) zumindest teilweise basierend auf dem Parameter des Atemgases oder zumindest teilweise basierend auf zumindest einem der folgenden Parameter einstellt; einem Druck des Atemgasstroms; einem Fluss oder Volumen des Atemgasstroms, eine absichtliche Leckage; ein unbeabsichtigte Leckage; eine Atemfrequenz; ein inspiratorisches Atemvolumen; ein exspiratorisches Volumen; ein I: E-Verhältnis; Beginn und Ende der Inspiration; Beginn und Ende der Exspiration; ein Spitzenfluss während der Inspiration; ein Spitzenflusses während der Exspiration; Umgebungstemperatur; Wassertemperatur, Luftfeuchtigkeit; eine Ausgangsleistung oder Zielleistung der Heizleistung; eine Übertragungsfunktion für die Heizleistung; ein Volumen des Schlauches; ein Volumen der Benutzerschnittstelle; Zeitdauer seit Therapiebeginn; Messwerte externer, drahtlos angebundener Sensoren, z. B. in einem Smartphone, oder anderweitiger externer Daten, z. B. Wetter-App im Smartphone.

Unterschiedliche Sensoren 21...29 erfassen in diesem Aufbau die Umgebungstemperatur, Umgebungsfeuchte, die Feuchte im Anfeuchter, die Wassertemperatur im Anfeuchter, den Atemgasstrom und die Abwärmeverluste im System und die Feuchte in der Maske. Insgesamt werden die relative Feuchte und die absolute Feuchte bestimmt. Die Heizleistung wird beispielsweise stufenlos anhand eines Kennfeldes, welches die benötigte Leistung bei bestimmten mittleren Gesamtflüssen aufschlüsselt, verändert.

Bevorzugt sind mehrere diskrete Stufen Heizleistung vorgebbar, wobei jeweils zumindest eine Kennlinie pro Stufe der Heizleistung abrufbar hinterlegt ist. Die Kennlinien können dabei basierend auf dem Aufbau der Figur 3 ermittelt worden sein.

Die Steuerung der Heizleistung erfolgt beispielsweise anhand von Korrektur-Parametern die hinterlegt sind, um Umgebungstemperaturen oder Luftfeuchte oder Wasser-Einfülltemperaturen auszugleichen.

Die Steuerung der Heizleistung erfolgt beispielsweise auch anhand von Daten eines Wasserstandssensors, sodass die Heizleistung bei geringem Wasserstand erniedrigt wird.

Im Sinne der Erfindung können beispielhafte Anfeuchter, wie sie hier beschrieben sind, Atemgas in Beatmungsgeräten befeuchten, indem eine Verdampfungsvorrichtung verwendet wird, die Wasser verdampft, wenn die Vorrichtung elektrisch betrieben wird.

Die Kombination der Steuerung basierend auf dem Atemfluss und/oder Leckage-Fluss (beabsichtigt, unbeabsichtigt) mit der Funktion des Temperaturanstiegs des Rampenmoduls ermöglicht eine komfortable Steuerung und Anfeuchtung des Atemgases. Erwartet der Anwender/Patient eine hohe Anfeuchtung der Atemluft bzw. stellt diese ein, so wird durch die Funktion des Temperaturanstiegs zu Beginn der Therapiesitzung/der Beatmung in einer ersten, zu der Gesamtdauer relativ kurzen Phase (z.B. maximal die ersten 30 Minuten) die Heizleistung des Heizelements auf eine hohe bzw. die höchste Stufe gesteuert. Dadurch wird ein schnelles Aufheizen des Wassers im Anfeuchter und somit ein schnelles Erreichen der gewünschten Anfeuchtung des Atemgases erreicht. Ist die gewünschte Anfeuchtung des Atemgases bzw. eine entsprechende Wassertemperatur erreicht, kann in der zweiten, im längeren Phase beispielsweise eine Steuerung der Heizleistung anhand des Gesamtflusses des Atemgases und/oder anhand Leckage-Flüssen erfolgen. Dazu sind beispielsweise Kennfelder hinterlegt, welche die benötigte Heizleistung mit dem aktuellen Fluss und der gewünschten Anfeuchtung des Atemgases in Relation setzten.

Die Figuren sind in erster Linie der Übersichtlichkeit halber wiedergegeben und daher nicht unbedingt maßstabsgetreu gezeichnet.

Darüber hinaus können verschiedene Strukturen / Komponenten, einschließlich, aber nicht beschränkt auf Befestigungselemente, elektrische Komponenten (Verdrahtung, Kabel usw.) und dergleichen, schematisch gezeigt oder aus einigen oder allen Ansichten entfernt werden, um Aspekte der dargestellten Ausführungsformen besser zu veranschaulichen, oder wo die Einbeziehung solcher Strukturen / Komponenten zum Verständnis der verschiedenen hierin beschriebenen beispielhaften Ausführungsformen nicht notwendig ist.

Das Fehlen einer Darstellung / Beschreibung solcher Strukturen / Komponenten in einer bestimmten Figur ist jedoch nicht als Einschränkung des Umfangs der verschiedenen Ausführungsformen in irgendeiner Weise zu interpretieren.

Der Begriff "Gas", wie er hier verwendet wird, umfasst fast jedes Gas oder jede Gas-Dampf-Kombination. Zum Beispiel kann das vom Gebläse bereitgestellte Gas Luft, Sauerstoff, Wasserdampf oder -tröpfchen, medizinischen Dampf oder medizinische Dämpfe und Kombinationen davon enthalten. Zur Vereinfachung der Beschreibung können die Ausdrücke "Luft", "Fluid" und "Gas" hierin austauschbar verwendet werden. "Druckgas", wie hierin verwendet, bezieht sich auf Gas mit einem positiven Druck im Verhältnis zum Umgebungsdruck. "Gebläse", wie hierin verwendet, bezieht sich auf die meisten Geräte oder Quellen, die einen Druckgasstrom erzeugen können.

**EP 3 858 415 B1**

**Patentansprüche**

1. Beatmungsgerät mit einer Atemgaseinheit (14) mit einem Anfeuchter (11), der ein Heizelement (13) und einen Wasserbehälter aufweist und zur Ankopplung an das Beatmungsgerät (1) ausgebildet ist, aufweisend:

   - zumindest einen Sensor (15), der Parameter des Atemgases erfasst;
   - zumindest eine Steuereinheit (16) zur Vorgabe der Heizleistung des Heizelementes (13) zumindest teilweise basierend auf dem Parameter des Atemgases, wobei die Steuereinheit zumindest ein Rampenmodul (33) aufweist,

   **dadurch gekennzeichnet, dass** das Rampenmodul (33)
   eine Funktion des Temperaturanstiegs im Anfeuchter (11) bereitstellt, damit die gewünschte Anfeuchtung oder die gewünschte Heizleistung schneller erreicht wird, und das in einer ersten, kürzer andauernden Phase eine höhere Heizleistung steuert als in einer zweiten, längeren Phase, wobei mehrere diskrete Stufen Heizleistung vorgebbar sind und jeweils zumindest eine Kennlinie pro Stufe der Heizleistung abrufbar hinterlegt ist, wobei die Vorgabe verschiedener Heizstufen durch das Rampenmodul (33) mittels Leistungsregelung realisiert wird, wobei durch eine zyklische Messung Strom und Spannung am Heizelement (13) erfasst werden.

2. Beatmungsgerät nach Anspruch 1, **dadurch gekennzeichnet, dass** die Steuereinheit (16) die Heizleistung des Heizelementes (13) zumindest teilweise basierend auf zumindest einem der folgenden Parameter einstellt: einem Druck, Fluss oder Volumen des Atemgasstroms; einer absichtlichen oder unbeabsichtigten Leckage; einer Atemfrequenz; einem inspiratorischen oder exspiratorischen Atemvolumen; einem I:E-Verhältnis; Beginn und Ende der Inspiration oder Exspiration; einem Spitzenfluss während der Inspiration oder Exspiration; Umgebungstemperatur; Wassertemperatur; Luftfeuchtigkeit; einer Ausgangsleistung oder Zielleistung der Heizleistung; einer Übertragungsfunktion für die Heizleistung; einem Volumen eines Schlauches; einem Volumen einer Benutzerschnittstelle; Zeitdauer seit Therapiebeginn; Messwerten externer, drahtlos angebundener Sensoren, z. B. in einem Smartphone, oder anderweitigen externen Daten, z. B. einer Wetter-App in einem Smartphone.

3. Beatmungsgerät nach Anspruch 1 oder 2, **dadurch gekennzeichnet, dass** der Sensor den Fluss des Atemgases ermittelt und die Steuereinheit (16) die Heizleistung des Anfeuchters entsprechend dem Fluss des Atemgases steuert.

4. Beatmungsgerät nach Anspruch 3, **dadurch gekennzeichnet, dass** die Steuereinheit (16) die Heizleistung des Anfeuchters zusätzlich entsprechend einer hinterlegten Übertragungsfunktion für die Heizleistung steuert.

5. Beatmungsgerät nach zumindest einem der vorhergehenden Ansprüche, **dadurch gekennzeichnet, dass** die Steuereinheit (16) einen Leckage-Fluss des Atemgases ermittelt und die Heizleistung des Anfeuchters entsprechend dem Leckage-Fluss des Atemgases steuert, wobei der Leckage-Fluss ein absichtlicher Leckage-Fluss und/oder ein unabsichtlicher Leckage-Fluss ist.

6. Beatmungsgerät nach zumindest einem der vorhergehenden Ansprüche, **dadurch gekennzeichnet, dass** die Heizleistung des Anfeuchters basierend auf dem mittleren Gesamtfluss derart gesteuert wird, dass die absolute Feuchte (Wassermenge pro Volumen) des abgegebenen Atemgases annähernd konstant bleibt, und/oder gesteuert wird, um die Austrocknung der Schleimhäute des Patienten bei hoher Leckage zu reduzieren und gleichzeitig zu verhindern, dass bei niedriger Leckage Wassertropfen im Beatmungsschlauch auskondensieren.

7. Beatmungsgerät nach zumindest einem der vorhergehenden Ansprüche, **dadurch gekennzeichnet, dass** die Steuereinheit (16) die Heizleistung des Anfeuchters so steuert, dass über einen Flussbereich vom 1 l/min bis 300 l/min ein konstantes Feuchtigkeitsniveau in dem angefeuchteten Atemgas erreicht wird.

8. Beatmungsgerät nach zumindest einem der vorhergehenden Ansprüche, **dadurch gekennzeichnet, dass** die Heizleistung stufenlos anhand eines Kennfeldes, welches die benötigte Leistung bei bestimmten mittleren Gesamtflüssen aufschlüsselt, verändert wird.

9. Beatmungsgerät nach zumindest einem der vorhergehenden Ansprüche, **dadurch gekennzeichnet, dass** zusätzlich eine Schlauchheizung umfasst ist, wobei die Schlauchheizung in Abhängigkeit vom Fluss oder Gesamtfluss oder von der Leckage gesteuert wird.

**10.** Beatmungsgerät nach zumindest einem der vorhergehenden Ansprüche, **dadurch gekennzeichnet, dass** der Anfeuchter (11) bei der Verbindung mit dem Beatmungsgerät (1) mit der Elektronik des Beatmungsgerätes gekoppelt und über das Beatmungsgerät (1) gesteuert wird.

**11.** Beatmungsgerät nach zumindest einem der vorhergehenden Ansprüche, **dadurch gekennzeichnet, dass** das Rampenmodul die Temperatur im Wasserbehälter und/oder die Umgebungstemperatur für die Steuerung der Heizleistung zumindest in der ersten Phase berücksichtigt und dazu Mittel (23) zum Erfassen der Wassertemperatur im Anfeuchter (11) angeordnet sind, um der Steuereinheit eine Ist-Temperatur zu übermitteln, wobei das Rampenmodul (33) unterschiedliche Leistungskurven abrufbar oder einstellbar vorgeben kann.

**12.** Beatmungsgerät nach zumindest einem der vorhergehenden Ansprüche, **dadurch gekennzeichnet, dass** das Rampenmodul (33) zusätzlich ein Verzögerungsmodul (34) beinhaltet, welches die Steuereinheit (16) zu einer einstellbar verzögerten Vorgabe der Heizleistung an das Heizelement (13) veranlasst, wobei das Verzögerungsmodul eine konkrete Zeitvorgabe, etwa in Minuten, ermöglicht und die Steuereinheit (16) zu einem einstellbar verzögerten Beginn einer Beatmungsfunktion veranlasst.

**13.** Beatmungsgerät nach zumindest einem der vorhergehenden Ansprüche, **dadurch gekennzeichnet, dass** die Leistung durch Pulsweitenmodulation nachgeregelt wird, damit auch bei einem sich ändernden Widerstand des Heizelementes eine gleichbleibende Leistungsabgabe ermöglicht wird.

**14.** Beatmungsgerät nach zumindest einem der vorhergehenden Ansprüche, **dadurch gekennzeichnet, dass** das Rampenmodul in der ersten, kürzer andauernden Phase die höhere oder die höchste Heizleistung steuert, wobei der Sensor den Fluss des Atemgases ermittelt und die Steuereinheit (16) die Heizleistung des Anfeuchters zumindest in der zweiten, längeren Phase entsprechend dem Fluss des Atemgases steuert.

**15.** Beatmungsgerät nach zumindest einem der vorhergehenden Ansprüche, **dadurch gekennzeichnet, dass** das Heizelement (13) eine temperaturabhängige Widerstandskennlinie aufweist und im Beatmungsgerät (1) der aktuelle Widerstandswert aus dem gemessenen Strom und der Spannung am Heizelement (13) errechnet wird, wobei der aktuelle Widerstandswert mit Schwellwerten verglichen wird zur Erkennung eines leeren Wasserbehälters und wobei bei Überschreitung eines Schwellwertes die Steuereinheit (16) die Heizleistung ausschaltet.

**Claims**

**1.** A ventilator having a respiratory gas unit (14) having a humidifier (11) which comprises a heating element (13) and a water container and is designed for coupling to the ventilator (1), comprising:

- at least one sensor (15) that records parameters of the respiratory gas;
- at least one control unit (16) for setting the heating power of the heating element (13) at least partially based on the parameter of the respiratory gas, wherein the control unit comprises at least one ramp module (33), **characterized in that** the ramp module (33) provides a function of the temperature rise in the humidifier (11), such that the desired humidification or the desired heating power is achieved more quickly, and which controls a higher heating power in a first, shorter phase than in a second, longer phase, wherein multiple discrete levels of heating power can be set and in each case at least one characteristic curve is stored per level of the heating power so as to be retrievable, wherein various heating levels are set by the ramp module (33) by means of closed-loop power regulation, wherein the current and voltage at the heating element (13) are detected by means of a cyclical measurement.

**2.** The ventilator according to claim 1, **characterized in that** the control unit (16) adjusts the heating power of the heating element (13) at least partially based on at least one of the following parameters: a pressure, flow, or volume of the respiratory gas stream; an intentional or unintentional leakage; a breathing rate; an inspiratory or expiratory breathing volume; an I:E ratio; start and end of inspiration or expiration; a peak flow during inspiration or expiration; ambient temperature; water temperature; air humidity; an output power or target power of the heating power; a transfer function for the heating power; a volume of a tube; a volume of a user interface; time since start of treatment; measured values from external, wirelessly connected sensors, e.g. in a smartphone, or other external data, e.g. from a weather app in a smartphone.

**3.** The ventilator according to claim 1 or 2, **characterized in that** the sensor determines the flow of the respiratory gas and the control unit (16) controls the heating power of the humidifier in accordance with the flow of the respiratory gas.

4. The ventilator according to claim 3, **characterized in that** the control unit (16) additionally controls the heating power of the humidifier in accordance with a stored transfer function for the heating power.

5. The ventilator according to at least one of the preceding claims, **characterized in that** the control unit (16) determines a leakage flow of the respiratory gas and controls the heating power of the humidifier in accordance with the leakage flow of the respiratory gas, wherein the leakage flow is an intentional leakage flow and/or an unintentional leakage flow.

6. The ventilator according to at least one of the preceding claims, **characterized in that** the heating power of the humidifier is controlled based on the average total flow in such a way that the absolute humidity (amount of water per volume) of the delivered respiratory gas remains approximately constant, and/or is controlled in order to reduce dehydration of the mucous membranes of the patient at a high leakage rate and, at the same time, in order to prevent water droplets from condensing in the ventilation tube at a low leakage rate.

7. The ventilator according to at least one of the preceding claims, **characterized in that** the control unit (16) controls the heating power of the humidifier such that a constant humidity level is achieved in the humidified respiratory gas over a flow range from 1 l/min to 300 l/min.

8. The ventilator according to at least one of the preceding claims, **characterized in that** the heating power is changed steplessly based on a characteristic map which breaks down the required power at particular average total flows.

9. The ventilator according to at least one of the preceding claims, **characterized in that** a tube heater is additionally comprised, wherein the tube heater is controlled depending on the flow or total flow or on the leakage.

10. The ventilator according to at least one of the preceding claims, **characterized in that** the humidifier (11) is coupled to the electronics of the ventilator when it is connected to the ventilator (1) and is controlled via the ventilator (1).

11. The ventilator according to at least one of the preceding claims, **characterized in that** the ramp module takes into account the temperature in the water container and/or the ambient temperature for the control of the heating power at least in the first phase and, for this purpose, means (23) for detecting the water temperature in the humidifier (11) are provided in order to transmit an actual temperature to the control unit, wherein the ramp module (33) can set different power curves so as to be retrievable or adjustable.

12. The ventilator according to at least one of the preceding claims, **characterized in that** the ramp module (33) additionally contains a delay module (34) which prompts the control unit (16) to set the heating power to the heating element (13) with an adjustable delay, wherein the delay module makes it possible to set a specific time, for example in minutes, and prompts the control unit (16) to start a ventilation function with an adjustable delay.

13. The ventilator according to at least one of the preceding claims, **characterized in that** the power is readjusted by means of pulse width modulation, such that a constant power output is possible even with a changing resistance of the heating element.

14. The ventilator according to at least one of the preceding claims, **characterized in that** the ramp module controls the higher or highest heating power in the first, shorter phase, wherein the sensor determines the flow of the respiratory gas and the control unit (16) controls the heating power of the humidifier at least in the second, longer phase in accordance with the flow of the respiratory gas.

15. The ventilator according to at least one of the preceding claims, **characterized in that** the heating element (13) has a temperature-dependent resistance characteristic curve and the current resistance value in the ventilator (1) is calculated from the measured current and voltage at the heating element (13), wherein the current resistance value is compared with threshold values in order to recognize an empty water container and wherein the control unit (16) switches off the heating power when a threshold value is exceeded.

**Revendications**

1. Appareil respiratoire avec une unité de gaz respiratoire (14) avec un humidificateur (11), lequel présente un élément chauffant (13) et un récipient d'eau, et est conçu pour l'accouplement à l'appareil respiratoire (1), présentant :

- au moins un capteur (15), lequel saisit des paramètres du gaz respiratoire ;
- au moins une unité de commande (16) pour la spécification de la puissance de chauffage de l'élément chauffant (13) basé au moins partiellement sur le paramètre du gaz respiratoire, l'unité de commande présentant au moins un module de rampe (33), **caractérisé en ce que** le module de rampe (33) fournit une fonction de l'augmentation de température dans l'humidificateur (11), afin que l'humidification souhaitée ou la puissance de chauffage souhaitée soient atteintes plus rapidement, et lequel règle une puissance de chauffage plus élevée dans une première phase, durant moins longtemps, que dans une deuxième phase, plus longue, plusieurs niveaux de puissance de chauffage discrets étant prédéfinissables et respectivement au moins une courbe caractéristique par niveau de la puissance de chauffage étant enregistrée de façon consultable, la spécification de différents niveaux de chauffage par le module de rampe (33) étant réalisée par réglage de puissance, le courant et la tension au niveau de l'élément chauffant (13) étant saisis à l'aide d'une mesure cyclique.

2. Appareil respiratoire selon la revendication 1, **caractérisé en ce que** l'unité de commande (16) règle la puissance de chauffage de l'élément chauffant (13) basé au moins partiellement sur au moins l'un des paramètres suivants : une pression, un débit ou un volume du flux de gaz respiratoire ; une fuite volontaire ou involontaire ; une fréquence respiratoire ; un volume respiratoire inspiratoire ou expiratoire ; un rapport I:E ; le début et la fin de l'inspiration ou de l'expiration ; un débit de pointe pendant l'inspiration ou l'expiration ; la température ambiante ; la température de l'eau ; l'humidité de l'air ; une puissance de sortie ou une puissance cible de la puissance de chauffage ; une fonction de transfert pour la puissance de chauffage ; un volume d'un tuyau ; un volume d'une interface utilisateur ; la durée depuis le début de la thérapie ; des valeurs de mesure de capteurs externes connectés sans fil, par exemple dans un smartphone, ou d'autres données externes, par exemple d'une appli météo dans un smartphone.

3. Appareil respiratoire selon la revendication 1 ou 2, **caractérisé en ce que** le capteur détermine le débit du gaz respiratoire et **en ce que** l'unité de commande (16) règle la puissance de chauffage de l'humidificateur corrélativement au débit du gaz respiratoire.

4. Appareil respiratoire selon la revendication 3, **caractérisé en ce que** l'unité de commande (16) règle en outre la puissance de chauffage de l'humidificateur corrélativement à une fonction de transfert enregistrée pour la puissance de chauffage.

5. Appareil respiratoire selon l'au moins une des revendications précédentes, **caractérisé en ce que** l'unité de commande (16) détermine un débit de fuite du gaz respiratoire et règle la puissance de chauffage de l'humidificateur corrélativement au débit de fuite du gaz respiratoire, le débit de fuite étant un débit de fuite volontaire et/ou un débit de fuite involontaire.

6. Appareil respiratoire selon au moins l'une des revendications précédentes, **caractérisé en ce que** la puissance de chauffage de l'humidificateur est réglée basé sur le débit total moyen de telle sorte que l'humidité absolue (quantité d'eau par volume) du gaz respiratoire délivré reste approximativement constante, et/ou est réglée afin de réduire l'assèchement des muqueuses du patient en cas de fuite élevée et simultanément d'empêcher que des gouttes d'eau se condensent dans le tuyau respiratoire en cas de fuite faible.

7. Appareil respiratoire selon au moins l'une des revendications précédentes, **caractérisé en ce que** l'unité de commande (16) règle la puissance de chauffage de l'humidificateur de telle sorte qu'un niveau d'humidité constant est atteint dans le gaz respiratoire humidifié sur une plage de débit de 1 l/min à 300 l/min.

8. Appareil respiratoire selon au moins l'une des revendications précédentes, **caractérisé en ce que** la puissance de chauffage est modifiée de façon continue à l'aide d'un diagramme caractéristique, lequel répartit la puissance requise pour des débits totaux moyens donnés.

9. Appareil respiratoire selon au moins l'une des revendications précédentes, **caractérisé en ce qu'**un chauffage de tuyau est compris en outre, le chauffage de tuyau étant commandé en fonction du débit ou du débit total ou de la fuite.

10. Appareil respiratoire selon au moins l'une des revendications précédentes, **caractérisé en ce que** l'humidificateur (11) est accouplé avec l'électronique de l'appareil respiratoire lors de la connexion avec l'appareil respiratoire (1) et est commandé par l'appareil respiratoire (1).

**11.** Appareil respiratoire selon au moins l'une des revendications précédentes, **caractérisé en ce que** le module de rampe prend en compte la température dans le récipient d'eau et/ou la température ambiante pour le réglage de la puissance de chauffage au moins dans la première phase et **en ce que**, en outre, des moyens (23) pour la saisie de la température de l'eau sont disposés dans l'humidificateur (11), afin de transmettre une température réelle à l'unité de commande, le module de rampe (33) pouvant spécifier des courbes de puissance différentes de façon consultable ou réglable.

**12.** Appareil respiratoire selon au moins l'une des revendications précédentes, **caractérisé en ce que** le module de rampe (33) comprend en outre un module de retard (34), lequel entraîne une spécification retardée de façon réglable de la puissance de chauffage à l'élément chauffant (13) par l'unité de commande (16), le module de retard permettant une spécification temporelle concrète, par exemple en minutes, et entraînant un début retardé de façon réglable d'une fonction respiratoire par l'unité de commande (16).

**13.** Appareil respiratoire selon au moins l'une des revendications précédentes, **caractérisé en ce que** la puissance est réglée de nouveau par modulation d'impulsions en largeur, afin qu'une puissance de sortie constante soit possible aussi en cas de résistance variable de l'élément chauffant.

**14.** Appareil respiratoire selon au moins l'une des revendications précédentes, **caractérisé en ce que** le module de rampe règle la puissance de chauffage plus élevée ou la plus élevée dans la première phase durant moins longtemps, le capteur déterminant le débit du gaz respiratoire et l'unité de commande (16) réglant la puissance de chauffage de l'humidificateur au moins dans la deuxième phase, plus longue, corrélativement au débit du gaz respiratoire.

**15.** Appareil respiratoire selon au moins l'une des revendications précédentes, **caractérisé en ce que** l'élément chauffant (13) présente une courbe caractéristique de résistance en fonction de la température et **en ce que**, dans l'appareil respiratoire (1), la valeur de résistance actuelle est calculée à partir du flux mesuré et de la tension au niveau de l'élément chauffant (13), la valeur de résistance actuelle étant comparée avec des valeurs seuils pour la détection d'un récipient d'eau vide et l'unité de commande (16) désactivant la puissance de chauffage en cas de dépassement d'une valeur seuil.

Fig. 1a

Fig. 1b

Fig. 2

Fig. 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20160067443 A1
- US 20190298962 A1
- WO 2004020031 A1

- US 20160193437 A1
- US 20190076617 A1
- WO 2016139645 A1